# EUROPEAN PATENT APPLICATION

(11) **EP 4 582 552 A1**
(43) Date of publication of application: **09.07.2025**
(21) Application number: 22956982.7
(22) Date of filing: 02.09.2022
(51) Int. Cl.: C12Q 1/68, C12N 15/11

(54) **DIFFERENTIAL METHYLATION REGION OF OPLAH GENE, KIT, AND USE**

(71) Applicant: BGI Genomics Co., Ltd., Yantian District Shenzhen, Guangdong Province 518083 (CN)
(72) Inventor: LI, Zhilong, Shenzhen, Guangdong 518083 (CN); WANG, Yuying, Shenzhen, Guangdong 518083 (CN); PENG, Jiaxi, Shenzhen, Guangdong 518083 (CN); JIANG, Ruijingfang, Shenzhen, Guangdong 518083 (CN); SUN, Jianlong, Shenzhen, Guangdong 518083 (CN)
(74) Representative: Inspicos P/S
(86) International application number: PCT/CN2022/116734
(87) International publication number: WO 2024/045160

(57) **Abstract**

The present application discloses a differential methylation region of the OPLAH gene, a kit, and use. Specifically, the differential methylation region is chr8:145106171-145107467. The differential methylation region of the present application can be used for effectively detecting colorectal cancer or precancerous lesions of colorectal cancer, or evaluating prognostic risks to colorectal cancer patients.

## Description

### Technical Field

The present invention belongs to the field of biomedicine, and relates to differentially methylated region of OPLAH gene, kit and use. Specifically, the kit is a kit for detecting the differentially methylated region. Specifically, the use is for detecting colorectal cancer, or for the prognostic risk assessment of patients with colorectal cancer.

### Background Art

Colorectal cancer occurring in the colon and rectum of the lower digestive tract is a common malignant tumor. According to the latest statistics, the number of new cases of colorectal cancer ranks third among men and second among women worldwide, and the number of deaths ranks fourth among men and third among women (Bray et al., 2018). In China, the prevention and control situation of colorectal cancer is also very severe, which ranks third among new cases of malignant tumors (388,000) and fifth among cases of deaths (187,000) (Zheng Rongshou et al., 2019). Colorectal cancer develops slowly, generally going through polyps, adenomas, and intestinal cancer, and it can take up to 5 to 10 years for adenoma to develop into intestinal cancer. If intervention is carried out in the early stages of colorectal cancer, the mortality rate can be significantly reduced. The five-year survival rate of patients with stage I colorectal cancer can reach more than 90%, while the five-year survival rate of patients with stage IV colorectal cancer is less than 20% (Marzieh Araghi et al., 2020).

Traditional colorectal cancer early screening technologies mainly include colonoscopy, fecal occult blood test, tumor markers CEA and CA19-9, etc. These technical means currently have certain limitations. Colonoscopy is the gold standard for colorectal cancer diagnosis. Lens and light source can be inserted from anus, move along cavity to pass through rectum, sigmoid colon and other parts to reach the end of ileum, and images can be transmitted to display in real time for the operating doctor to observe. The images of colonoscope are intuitive and clear, and various lesions such as cancer, polyps, ulcers, and bleeding can be observed. Polyps can also be removed under microscope. However, it is an invasive detection technology with a complicated preparation process, requiring diet control and intestinal cleaning, and the compliance of examinees is low. In addition, it also requires equipment and personnel, and needs to be operated by hospital professionals and anesthesiologists. Some examinees will feel uncomfortable and have the risk of complications (3 to 5 cases/1000 people). Fecal occult blood test is a non-invasive method that detects blood components (hemoglobin) in stool to determine whether there is gastrointestinal bleeding, so that doctors can judge the risk of colorectal cancer. This method is quick and simple, and the compliance of examinees is high. However, this detection method is easily affected by liver, blood products, green leafy vegetables, etc. in the diet, resulting in false positives. At the same time, it has low sensitivity for early colorectal cancer patients with less bleeding, and is prone to missed diagnosis. CEA and other broad-spectrum tumor markers have low specificity, are prone to more false positives, and have limited sensitivity. Therefore, it is in need to establish an accurate, simple and economical early screening method for colorectal cancer.

DNA methylation is an important gene expression regulation mechanism that can regulate gene expression and silencing, and has a significant impact on the occurrence and development of tumors. Abnormal methylation of cancer-related genes often occurs in the early stages of cancer, so DNA methylation signals are considered to be potential early screening markers for tumors. Human fecal samples are mixed with intestinal cell DNA, which carries information such as methylation. By detecting and analyzing this information, the possibility of examinee suffering from colorectal cancer can be inferred. Some genes have been proven to be methylation markers for colorectal cancer detection in feces, such as SFRP2, SEPT9, NDRG4 and SDC2 (Hannes M Müller et al., 2004; Jie Chen et al., 2019), but their performance cannot fully meet clinical needs. For example, the sensitivity and specificity of SFRP2 for colorectal cancer are only 77% (Hannes M Müller et al., 2004).

The OPLAH gene encodes 5-hydroxyproline enzyme and affects the glutamate salvage pathway and abnormal expression. Studies have shown that the downregulation of expression caused by abnormal hypermethylation of OPLAH affects the metabolism of glutathione and is accompanied by tumorigenesis (Liu Y et al., 2014).

At present, there is still a need to develop new markers for colorectal cancer detection.

### Contents of the present invention

After in-depth research and creative work, the inventors discovered the differentially methylated region (DMR) of the OPLAH gene. The inventors surprisingly found that the differentially methylated region can be used to effectively detect a colorectal cancer or colorectal cancer precancerous lesions, or to assess the prognostic risk of colorectal cancer patients, with good sensitivity and specificity. The present invention is provided as follows:
One aspect of the present invention relates to a differentially methylated region, as follows:
chr8: 145106171-145107467 (also referred to as the DMR of the present invention).

The differentially methylated region is a differentially methylated region of OPLAH gene.

In some embodiments of the present invention, the differentially methylated region has a base sequence as set forth in SEQ ID NO: 1 or a complementary sequence thereof.

Base sequence of DMR:

CGCCGTAGCTGCGTCCCCAGAACCGGGAGACTTAAGGCATCTTTATTGCGGGAT
CCTCACACGGCCTCCTGGGCCCGGCGATACTCATAGACGCTGCCGTGCTCGGGAAA
GGCCAGTGCTTGCGGGGGCGACCCCGGCGGTGGGGCGGGGTCCTCCGGGTCCCCAT
AGCCACCGCCGCCGGGCGTGTGGAGACAGAACACATCCTGTTGGCGCGGGGGGGGG
CGGGGAGGCGGGCTCAGTGCAGGCGTGGCCCCTCCCTGTCACCTGCGCAGTCCCCTC
CCCACCGGGCAGCGTCCATCACCCGCCCCCATGGACCACGGAGGCCCGGTACGCGC
CCCCTTTCCTTCCGGGGCCAAATTAAACTCGGCCTCTGGTCAGGTCTGCAACTGTTCC
CCCTCTGTGGGATGGGGCCGGGAGGGGAGGGGAGGGGAGGGGGACAGGACAGGCC
GCGGCCCTTACCCCGGGGTACACGGTCACCGACGTCTTGCCGCCCAGATTCACCGTC
CGGCCGTTTTTGCGGATCAGCAGGTTTAGGCCGCGGGCGCCAGGCTCGCCCCCTGCG
GAGGGAGGCGAGGAGTCCAGAGAGACCAGGGGCGGGGGTGGGGGCGGGGGCGGG
GAGGGCCGCGAGGGCTGGGGAACCGCGCACCGTGGAGCCCGTATGGCCGGAAGGC
GCGGCGCTCGGTCAGCACTGACAGCAGCGCCTCCTCACGAAAGAGCAGCTCGCGGG
TGACGCCGTCGCCGCCTCGGAAGCGGCCTCTGCCCCCCGAGCCCCCCGCCGCAGCTC
GAAGCGGCGCAGGATGACCGGGTACCTGCGAGGGCGAGGACGAGGGCAAAGACTC
AGCGTGGCCCGAGCCCCGGCTCCCACCCGGCCGTGCCCCCAGCCTCCGCGCACGCC
GCTCACCGGCTCTCCAGGATCTCAGGGTCGGTGATGCGTGTGTTGGTCATGTGGCTG
TGCACACCGCTGCGCCCGTGCCAGCTGGGACCCGCGCCCGCGCCGCCCGCCACCGTC
TCGTAGTAGCCCATGTGGGCGTTGCCCAGGGTCACGTTGTTCATGCAGCCCTGGTGA
GGGCACCTGGTCGCTGCGCTGGGCTGGGGCAGGGCGTCCCCACCTCGCCCTCCCGCT
CCTACTCCAGCCTGCCCACCCAGTCCGCCCCCGAGCTGCGCCCACCCCGCCCCCGCA
CCTGGGAGGCGGCGCAGGCCCCAAAGGCCCCCAGGATGACATCCACCACGCGCTGC
GACGTGAGCACGTTGCCGCCCACCACCGCCGCCTCGGGCGACGGGTCCAGGATGGA
GCCTC (SEQ ID NO: 1)

In some embodiments of the present invention, the differentially methylated region is used to detect a colorectal cancer or colorectal cancer precancerous lesions, or to assess the prognostic risk of a colorectal cancer patient.

In some embodiments of the present invention, the differentially methylated region has been treated as follows:
bisulfite treatment, methylation sensitive endonuclease treatment, methylation restriction endonuclease treatment or methylation modification enzyme treatment.

The differentially methylated region of the present invention is an OPLAH methylation marker for colorectal cancer detection.

Another aspect of the present invention relates to a kit, comprising a reagent for detecting the differentially methylated region as described in any one of items of the present invention or a reagent for detecting a methylation site within the differentially methylated region.

In some embodiments of the present invention, in the kit, the reagent comprises:
(1) a primer and a probe for detecting the differentially methylated region or the methylation site within the differentially methylated region;
   preferably, the reagent further comprises:
   (2) a primer and a probe for an internal reference gene.

In some embodiments of the present invention, in the kit:
in (1), the sequence of the primer is selected from SEQ ID NOs: 2 to 78, and the sequence of the probe is selected from SEQ ID NOs: 79 to 107;
preferably, in (1): the sequence of the primer is selected from SEQ ID NOs: 29 and 31, and the sequence of the probe is selected from SEQ ID NO: 89; the sequence of the primer is selected from SEQ ID NOs: 24 to 25, and the sequence of the probe is selected from SEQ ID NO: 83; the sequence of the primer is selected from SEQ ID NOs: 25 to 26, and the sequence of the probe is selected from SEQ ID NO: 87; the sequence of the primer is selected from SEQ ID NOs: 32 to 33, and the sequence of the probe is selected from SEQ ID NO: 90; or the sequence of the primer is selected from SEQ ID NOs: 55 to 56, and the sequence of the probe is selected from SEQ ID NO: 103;
preferably, in (2), the sequence of the primer of the internal reference gene is set forth in SEQ ID NOs: 108 to 109, and the sequence of the probe of the internal reference gene is set forth in SEQ ID NO: 110.

In some embodiments of the present invention, the kit further comprises one or more selected from the following items:
a stool sample sampler, a nucleic acid extraction reagent, a methylation detection sample pretreatment reagent, and a PCR reagent.

In some embodiments of the present invention, in the kit, the methylation detection sample pretreatment reagent is a bisulfite, a methylation-sensitive endonuclease, a methylation restriction endonuclease, or a methylation modification enzyme.

In some embodiments of the present invention, the kit further comprises PCR primers and/or probes for detecting one or more genes selected from the following:
ZNF132, TLX2, TFPI2, LONRF2, ITGA4, and ARHGEF4.

The protein encoded by ZNF132 gene belongs to the C2H2 zinc finger protein family and is involved in the biological development and differentiation process. Studies have shown that zinc finger proteins play a role in cancer progression (Hajra, K. M. et al., 2002).

TLX2 encodes a key factor in the development of enteric nervous system. Studies have shown that TLX2 functional loss may play a role in the occurrence of gastrointestinal stromal tumors (Kaifi JT et al., 2006).

TFPI2 gene encodes a member of the Kunitz family of serine protease inhibitors. Studies have shown that TFPI2 plays a role in maintaining tumor stability and inhibiting tumor growth (Ewa Sierko et al., 2007).

LONRF2 encodes the N-terminal domain of LON peptidase and RING finger protein 2. Studies have shown that LONRF2 may play an important role in the occurrence of rectal adenocarcinoma (Yang Hua et al., 2017).

ITGA4 gene encodes a protein that is a member of the integrin α chain family. It is a signaling molecule involved in tumor cell movement (Leila Darzi et al., 2017).

ARHGEF4 is a functional linker protein that connects tumor suppressor APC and G protein signal, and plays a role in the occurrence of colon cancer (Y Kawasaki et al., 2000).

Another aspect of the present invention relates to the use of the differentially methylated region of the present invention or the reagent for detecting the differentially methylated region or the methylation site in any one of the differentially methylated regions of the present invention in the manufacture of a medicament for detecting a colorectal cancer or a colorectal cancer precancerous lesion, or for assessment of the prognostic risk of a colorectal cancer patient.

In some embodiments of the present invention, in the use, the reagent is a primer and a probe for detecting the differentially methylated region or the methylation site in the differentially methylated region as described in any one of items of the present invention.

In some embodiments of the present invention, in the use, the primer and the probe are the primer and the probe as described in the above item (1).

In some embodiments of the present invention, in the use, the reagent further comprises a primer and a probe of an internal reference gene; preferably, the sequence of the primer of the internal reference gene is set forth in SEQ ID NOs: 108 to 109, and the sequence of the probe of the internal reference gene is set forth in SEQ ID NO: 110.

Another aspect of the present invention relates to a method for detecting a colorectal cancer or a colorectal cancer precancerous lesion or a method for assessing the prognostic risk of a colorectal cancer patient, comprising the following steps:
detecting the content of the differentially methylated region as described in any one of items of the present invention or the content of a fragment thereof; wherein the fragment comprises one or more methylation sites, for example, 1 to 20, 1 to 15, 1 to 10, 1 to 5, 1, 2, 3, 4 or 5 methylation sites, in the differentially methylated region.

Those skilled in the art will appreciate that the detected content of the fragment in the differentially methylated region, for example, by PCR, can represent the content of the differentially methylated region.

In some embodiments of the present invention, in the method, the content of the differentially methylated region or the fragment thereof as described in any one of items of the present invention is detected by a fluorescent quantitative PCR method.

In some embodiments of the present invention, in the method,
an internal reference gene Ct value is used to evaluate the content of human DNA in a sample; when the internal reference gene Ct value is >37, the sample human DNA content is judged to be too low and the detection fails; when the internal reference gene Ct value is ≤37, the sample is evaluated to be qualified and can be analyzed later.

In some embodiments of the present invention, in the method,
if the Ct value of the differentially methylated region is ≤35 or if △Ct is ≤ a cutoff value (for example, the cutoff value is 5), it is determined that the risk of colorectal cancer is high, otherwise it is determined that the risk is low;
wherein △Ct = (the Ct value of the differentially methylated region or the Ct value of an amplified fragment) - the Ct value of the reference gene;
wherein, the amplified fragment contains one or more methylation sites, for example, 1 to 20, 1 to 15, 1 to 10, 1 to 5, 1, 2, 3, 4 or 5 methylation sites, in the differentially methylated region.

In some embodiments of the present invention, in the method, the sample is a human tissue sample, a blood sample, a cell sample, a secretion sample or an excrement sample such as a stool sample.

According to some embodiments of the present invention, a stool sample is used as a detection object. In this way, the stool sample contains intestinal exfoliated cells, meets the requirements of the detection sample, is non-invasive, and is more patient-friendly.

According to some embodiments of the present invention, the optional detection means include but are not limited to: Sanger sequencing, pyrophosphate sequencing, high-throughput sequencing, nucleic acid mass spectrometry, PCR, fluorescence quantitative PCR, ddPCR, etc. Since the nucleic acid sequence has not changed, methylation modification is difficult to be directly detected, the nucleic acid often needs to be pretreated during the experiment. The pretreatment means herein include but are not limited to: bisulfite treatment, methylation sensitive endonuclease treatment, methylation restriction endonuclease treatment or methylation modification enzyme treatment. At the same time, the third-generation sequencing technology has been proven to be useful to directly determine the methylation modification state of DNA. With the development of technology, it can also be used as a detection means in the future. Fluorescence quantitative PCR technology is one of the commonly used and mature detection technologies for molecular diagnosis, which has the characteristics of rapidity, accuracy and low cost. In the preferred embodiment of the present invention, a method of bisulfite treatment combined with fluorescence quantitative PCR is adopted, and several methylation-specific PCR primers and probes are designed for the OPLAH gene, which can specifically identify methylated DNA molecules, realize the detection of the methylation state of the sample, and then evaluate the cancer risk of the subject.

By performing whole genome methylation sequencing and targeted methylation sequencing on colorectal cancer tissue and control tissue samples, we obtained the methylation difference regions highly associated with colorectal cancer, among which the methylation rate difference of region chr8: 145106171-145107467 was particularly significant. Primers and probes were designed for the region, which can be used to quickly and accurately detect the methylation status of the OPLAH gene. The present invention provides several primer and probe sequences, as shown in Table 1:

**Table 1: OPLAH gene primers, probes**

| SEQ ID NO: | Sequence (5'-3') | Description |
|---|---|---|
| 2 | GAATTAGGCGTCGTAGTTGC | OPLAH gene primers |
| 3 | CGCAAACACTAACCTTTCCC | |
| 4 | GGGTTCGGCGATATTTATAGAC | OPLAH gene primers |
| 5 | CCCCACCGCCGAAATC | |
| 6 | AGTACGGTAGCGTTTATGAGTATC | OPLAH gene primers |
| 7 | AACTAAACGCCGTAACTACG | |
| 8 | TTATCGTCGGGGTCGTTTTC | OPLAH gene primers |
| 9 | ACATCTTTATTACGAAATCCTCACAC | |
| 10 | TCGTCGGGTTTAGGAGGTC | OPLAH gene primers |
| 11 | ACTCGAAACACGAACTAAACG | |
| 12 | TATTTTTATTGCGGGATTTTTATAC | OPLAH gene primers |
| 13 | CAAAATATATTCTATCTCCACACGC | |
| 14 | CGTAGTTGCGTTTTTAGAATCG | OPLAH gene primers |
| 15 | TATAAAAACCCGAAAAACCCCG | |
| 16 | GGGTAGTGCGTTTGTAGTTTC | OPLAH gene primers |
| 17 | CGACAACGTCTATAAATATCGC | |
| 18 | TTTTGGAGGGGTTTACGGATTATC | OPLAH gene primers |
| 19 | CCAAAAAACCGTATAAAAATCCCG | |
| 20 | AAGGTAAGTATAGATGAGGGGCG | OPLAH gene primers |
| 21 | CGCAAACACTAACCTTTCCCG | |
| 22 | GAGGTCGTGTGAGGATTTCG | OPLAH gene primers |
| 23 | AACAATACGCCTACAACTCCG | |
| 24 | GGGTAGTGCGTTTGTAGTTTCG | OPLAH gene primers |
| 25 | ACACGACAACGTCTATAAATATCGC | |
| 26 | TCGGGTAGTGCGTTTGTAGTTTC | OPLAH gene primers |
| 25 | ACACGACAACGTCTATAAATATCGC | |
| 27 | GGTTTACGGATTATCGGGTAGTGC | OPLAH gene primers |
| 28 | CGACAACGTCTATAAATATCGCCG | |
| 29 | GGGCGTTGTTTAGGGTTAC | OPLAH gene primers |
| 30 | AAACGCAACTCGAAAACG | |
| 29 | GGGCGTTGTTTAGGGTTAC | OPLAH gene primers |
| 31 | CCCTACCCCAACCCAAC | |
| 32 | CGTTCGTGTTAGTTGGGATTC | OPLAH gene primers |
| 33 | CATAAACAACGTAACCCTAAACAAC | |
| 34 | GGGACGTTTTGTTTTAGTTTAGC | OPLAH gene primers |
| 35 | CCCGTACCAACTAAAACCC | |
| 36 | TATTTGCGAGGGCGAGGAC | OPLAH gene primers |
| 37 | CCTAAAAAACCGATAAACGACG | |
| 38 | CGTTCGTGTTAGTTGGGATGC | OPLAH gene primers |
| 33 | CATAAACAACGTAACCCTAAACAAC | |
| 32 | CGTTCGTGTTAGTTGGGATTC | OPLAH gene primers |
| 39 | TAAACAACGTAACCCTAAACACCG | |
| 40 | GGGCGTTGTTTAGGGTTGC | OPLAH gene primers |
| 31 | CCCTACCCCAACCCAAC | |
| 29 | GGGCGTTGTTTAGGGTTAC | OPLAH gene primers |
| 41 | CCTACCCCAACCCACCG | |
| 42 | CGTTCGTGTTAGTTGGGCTTC | OPLAH gene primers |
| 33 | CATAAACAACGTAACCCTAAACAAC | |
| 32 | CGTTCGTGTTAGTTGGGATTC | OPLAH gene primers |
| 43 | TAAACAACGTAACCCTAAACCACG | |
| 44 | CGTTCGTGTTAGTTGGGATCC | OPLAH gene primers |
| 33 | CATAAACAACGTAACCCTAAACAAC | |
| 45 | CGTTCGTGTTAGTTGGGGTTC | OPLAH gene primers |
| 33 | CATAAACAACGTAACCCTAAACAAC | |
| 32 | CGTTCGTGTTAGTTGGGATTC | OPLAH gene primers |
| 46 | TAAACAACGTAACCCTAAACGACG | |
| 32 | CGTTCGTGTTAGTTGGGATTC | OPLAH gene primers |
| 47 | TAAACAACGTAACCCTAAACAACG | |
| 29 | GGGCGTTGTTTAGGGTTAC | OPLAH gene primers |
| 48 | CCTACCCCAACCCAACG | |
| 49 | AAGCGGCGTAGGATGATC | OPLAH gene primers |
| 50 | CCGAAACTCGAACCACG | |
| 49 | AAGCGGCGTAGGATGATC | OPLAH gene primers |
| 51 | AAACCGAAACTCGAACCAC | |
| 32 | CGTTCGTGTTAGTTGGGATTC | OPLAH gene primers |
| 52 | TTTAAAACCTACGCCGCCT | |
| 32 | CGTTCGTGTTAGTTGGGATTC | OPLAH gene primers |
| 53 | ACGAAATAAAAACGCCCTACC | |
| 32 | CGTTCGTGTTAGTTGGGATTC | OPLAH gene primers |
| 30 | AAACGCAACTCGAAAACG | |
| 32 | CGTTCGTGTTAGTTGGGATTC | OPLAH gene primers |
| 54 | ACTCGAAAACGAACTAAATAAACAAACT | |
| 55 | TGAATAACGTGATTTTGGGTAACG | OPLAH gene primers |
| 56 | CGACTCTCCAAAATCTCAAAATCG | |
| 57 | GGGTAAAGATTTAGCGTGGTTC | OPLAH gene primers |
| 47 | TAAACAACGTAACCCTAAACAACG | |
| 32 | CGTTCGTGTTAGTTGGGATTC | OPLAH gene primers |
| 31 | CCCTACCCCAACCCAAC | |
| 32 | CGTTCGTGTTAGTTGGGATTC | OPLAH gene primers |
| 58 | ACGAAATAAACGCAACTCG | |
| 32 | CGTTCGTGTTAGTTGGGATTC | OPLAH gene primers |
| 59 | CGCAACTCGAAAACGAACT | |
| 60 | AGGATGATCGGGTATTTGCG | OPLAH gene primers |
| 61 | CAACCACATAACCAACACACG | |
| 60 | AGGATGATCGGGTATTTGCG | OPLAH gene primers |
| 62 | GCGAATCCCAACTAACACG | |
| 63 | GACGAGGGTAAAGATTTAGCG | OPLAH gene primers |
| 62 | GCGAATCCCAACTAACACG | |
| 64 | GGTAAAGATTTAGCGTGGTTCG | OPLAH gene primers |
| 47 | TAAACAACGTAACCCTAAACAACG | |
| 65 | TCGGTTTTTTAGGATTTTAGGGTCG | OPLAH gene primers |
| 66 | CCTCACCAAAACTACATAAACAACG | |
| 67 | TTGTATGAATAACGTGATTTTGGGTAACG | OPLAH gene primers |
| 68 | CACCGACTCTCCAAAATCTCAAAATCG | |
| 69 | TTATCGATTTTGAGATTTTGGAGAGTCG | OPLAH gene primers |
| 70 | CTCGATCAACACTAACAACAACG | |
| 71 | GCGTAGGATGATCGGGTATTTGC | OPLAH gene primers |
| 72 | CGAATCCCAACTAACACGAACG | |
| 73 | TTTGCGGAGGGAGGCG | OPLAH gene primers |
| 74 | GCGATTCCCCAACCCTCG | |
| 75 | GGAGTTTAGAGAGATTAGGGGCG | OPLAH gene primers |
| 76 | CGACCATACGAACTCCACG | |
| 77 | TCGTGGAGTTCGTATGGTCG | OPLAH gene primers |
| 78 | CCCGCGAACTACTCTTTCG | |
| 79 | ACACGACAACGTCTATAAATATCGCCGAACC | OPLAH gene probe |
| 80 | CCCGACGATACTCATAAACGCTACCGTA | OPLAH gene probe |
| 81 | CCGTAACTACGTCCCCAAAACCGAAA | OPLAH gene probe |
| 82 | TCGGGTTTAGGAGGTCGTGTGAGG | OPLAH gene probe |
| 83 | AACCGTATAAAAATCCCGCAATAA | OPLAH gene probe |
| 84 | AAACGCAACTACGACGCCTAATTCGTAC | OPLAH gene probe |
| 85 | TTGGCGCGTATAGATACGATTCGGAGT | OPLAH gene probe |
| 86 | TAGTGCGTTTGTAGTTTCGAGT | OPLAH gene probe |
| 87 | CTCCCGATTCTAAAAACGCAA | OPLAH gene probe |
| 88 | ACGTAGTTACGGCGTTT | OPLAH gene probe |
| 89 | CAACGACCAAATACCCTCA | OPLAH gene probe |
| 90 | TTCGCGTCGTTCGTTATCGTTT | OPLAH gene probe |
| 91 | AGCGTGGTTCGAGTTTCGGTTT | OPLAH gene probe |
| 92 | ATTTGGTCGTTGCGTTGGGTTGG | OPLAH gene probe |
| 93 | CCCGCCACCGTCTCGTAAT | OPLAH gene probe |
| 94 | TTGCGAGGGCGAGGACGAGG | OPLAH gene probe |
| 95 | CGAGGACGAGGGTAAAGATTTAGCGT | OPLAH gene probe |
| 96 | AACGCAACGATATACACAACCACATAACCAAC | OPLAH gene probe |
| 97 | TTCGAGTTTCGGTTTTTATTCGGTCGTGT | OPLAH gene probe |
| 98 | TTCGGTTTTTATTCGGTCGTG | OPLAH gene probe |
| 99 | CACCGACCCTAAAATCCTAAAAAACCGAT | OPLAH gene probe |
| 100 | ACCGATAAACGACGTACGCGAAAACT | OPLAH gene probe |
| 101 | CTACGAAACGATAACGAACGACGCGAA | OPLAH gene probe |
| 102 | ACGAGGGTAAAGATTTAGCGT | OPLAH gene probe |
| 103 | CACACCGCTACGCCCGTACCAA | OPLAH gene probe |
| 104 | TGAGCGGCGTGCGCGGAG | OPLAH gene probe |
| 105 | AGTTTAGAGAGATTAGGGGCG | OPLAH gene probe |
| 106 | TACGCGATTCCCCAACCCTCGCGAC | OPLAH gene probe |
| 107 | AAGGCGCGGCGTTCGGTTAG | OPLAH gene probe |

After pretreatment, human biological samples can be used to specifically amplify methylated nucleic acid fragments using optimized primers, probes and PCR reaction systems. Obvious amplification bands appear in highly methylated samples, while weak or no amplification occurs in lowly methylated samples. The methylation status of the sample can be determined by the Ct value and curve. For the detection of OPLAH methylation status, reference gene primers and probes including but not limited to those described in Table 2 can also be added to detect human DNA in the sample to obtain the corresponding Ct value, and the methylation status of the sample can be determined by calculating the difference (△Ct) between the OPLAH gene Ct value and the reference gene Ct value. Furthermore, by detecting samples from several cancer patients and non-cancer patients, a cutoff value (also called a positive judgment value, which can be obtained by drawing an ROC curve, for example) for determining the risk of cancer can be obtained. By comparing the detection results with the cutoff value, the risk of suffering cancer in the subject can be evaluated.

**Table 2: Sequence table of internal reference gene primers and probes**

| SEQ ID NO: | Sequence (5'-3') | Description |
|---|---|---|
| 108 | TTTAGGAGTGAGTGGAAGATAGA | Internal reference gene primers |
| 109 | AAACCACACCATCCTAATTACCT | |
| 110 | CCCAAAACACATTTCTTCCATTC | Internal reference gene probe |

The optimized primers, probes and PCR reaction system can form an OPLAH gene methylation detection kit, and the kit can be further used for cancer risk assessment.

Another aspect of the present invention relates to the use of the differentially methylated region of the present invention in preparing a product for detecting a colorectal cancer or a colorectal precancerous lesion.

In the present invention, if not otherwise specified, the term "colorectal cancer" refers to colon cancer and/or rectal cancer, also briefly referred to as "intestinal cancer".

In the present invention, if not otherwise specified, the term "early colorectal cancer" refers to stage I or stage II colorectal cancer.

In the present invention, if not otherwise specified, detecting a differentially methylated region or detecting a fragment thereof refers to detecting the content of a differentially methylated region or detecting the content of a fragment thereof; wherein the fragment comprises one or more methylation sites within the differentially methylated region.

In the present invention, if not otherwise specified, detecting a methylation site within a differentially methylated region refers to detecting the content of a methylation site within a differentially methylated region or detecting the methylation rate of a methylation site within a differentially methylated region.

### Beneficial Effects of the Invention

The present invention achieves one or more of the following technical effects:
(1) capable of realizing rapid detection of the methylation status of OPLAH gene;
(2) capable of detecting, diagnosing or screening colorectal cancer, or being applied to the prognostic risk assessment of colorectal cancer, with high sensitivity and/or specificity; preferably, with both high sensitivity and specificity;
(3) capable of being applied to the detection, diagnosis or screening of early colorectal cancer or colorectal precancerous lesions, with high sensitivity and/or specificity; preferably, with both high sensitivity and specificity.

### Brief Description of the Drawings

Figure 1 shows the differentially methylated gene region of colorectal cancer.
Figure 2A to Figure 2G show the performance comparison of colorectal cancer-specific methylation genes in colorectal cancer detection, wherein:

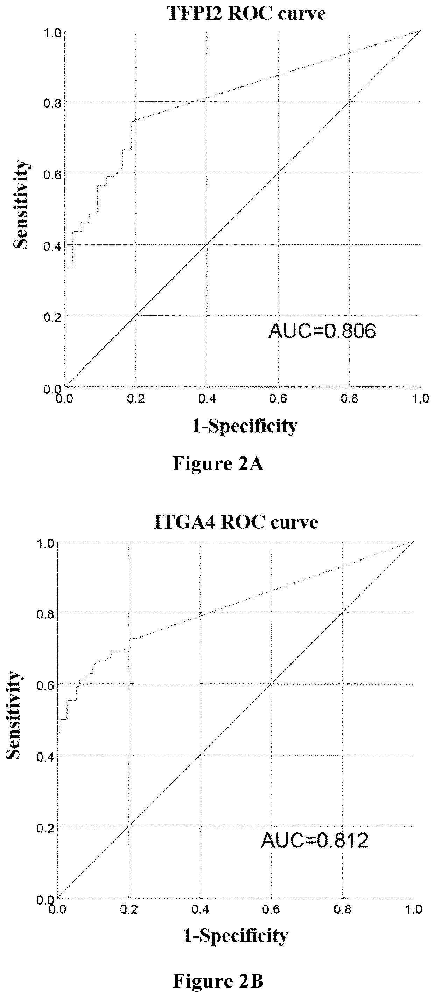

Figure 2A shows the ROC curve of TFPI2 gene detection of clinical samples;
Figure 2B shows the ROC curve of ITGA4 gene detection of clinical samples;

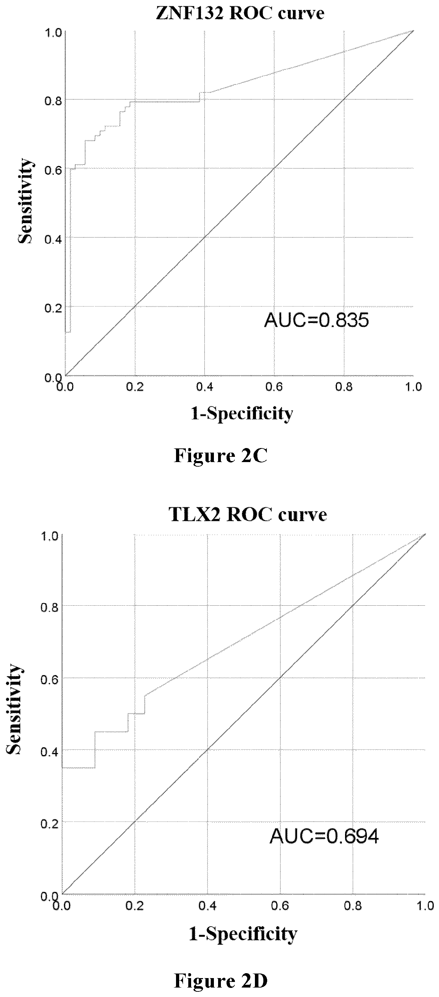

Figure 2C shows the ROC curve of ZNF132 gene detection of clinical samples;
Figure 2D shows the ROC curve of TLX2 gene detection of clinical samples;

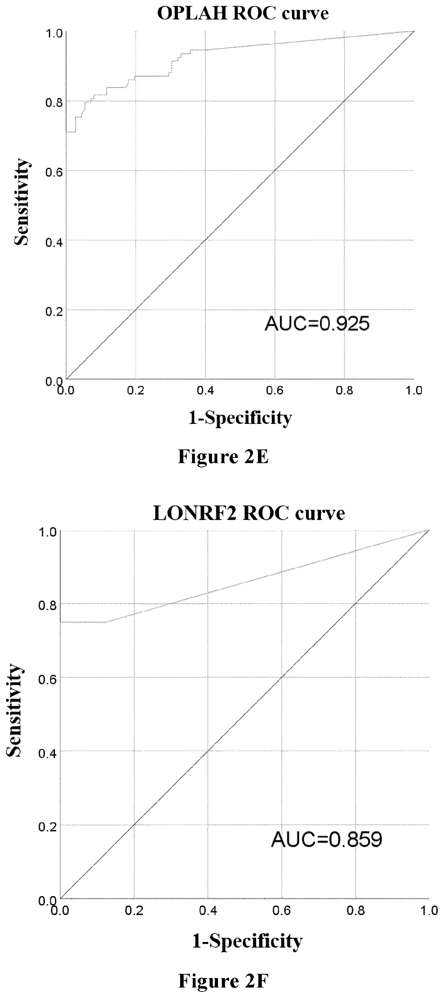

Figure 2E shows the ROC curve of OPLAH gene detection of clinical samples;
Figure 2F shows the ROC curve of LONRF2 gene detection of clinical samples;

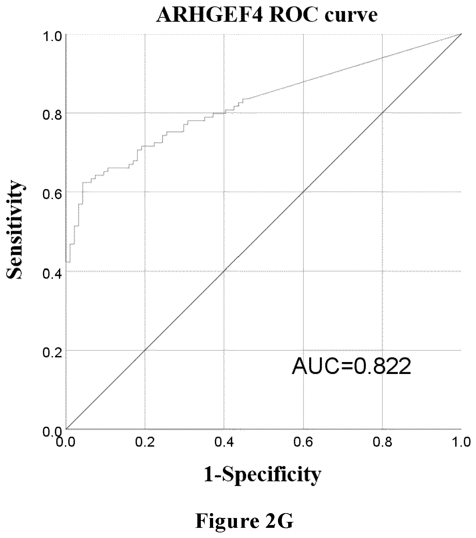

Figure 2G shows the ROC curve of ARHGEF4 gene detection of clinical samples.

### Specific Models for Carrying Out the present invention

The technical solution of the present invention will be described in detail in conjunction with the examples, but those skilled in the art will understand that the following examples are only used to illustrate the present invention and should not be regarded as limiting the scope of the present invention. If the specific conditions were not specified in the examples, they were carried out according to conventional conditions or conditions recommended by the manufacturer. The reagents or instruments used without indicating the manufacturer were all conventional products that could be purchased commercially.

Some relevant definitions refer to the Colorectal Cancer Diagnosis and Treatment Guidelines 2021, the Chinese Society of Clinical Oncology (CSCO), and some relevant contents are quoted as follows in Table 3:

**Table 3**

| | |
|---|---|
| Colorectal cancer | Colon cancer or rectal cancer confirmed by biopsy pathological diagnosis. |
| Advanced adenoma | Carcinoma in situ and intramucosal carcinoma, with diameter >= 1 cm, or with villous structure, or with high-grade neoplasia, with severe dysplasia. |
| Adenoma patients | Patients with adenoma confirmed by pathological diagnosis. |
| Polyp patients | Patients with inflammatory polyps or hyperplastic polyps confirmed or unconfirmed by pathological diagnosis. |
| Other intestinal disease patients | Including patients with non-polyp lesions such as enteritis, diverticulum, and colon melanosis. |

The standard for staging colorectal cancer adopts AJCC.

### Example 1: Discovery of DMR of colorectal cancer-specific methylated genes

### 1. Extraction of tissue samples:

DNeasy Blood & Tissue Kit (Qiagen, #69506) was used to extract DNA from 38 pairs of colorectal cancer sample and para-cancerous tissue sample from 38 people, wherein each pair of colorectal cancer sample and para-cancerous tissue sample came from the same person. Quantification was performed using the Qubit 3.0 system (Invitrogen, USA).

### 2. DNA fragmentation:

200 ng of the extracted DNA was taken, and added with 1 ng of Unmethylated lambda DNA (PROMEGA, #D1521) for subsequent C-U conversion quality control. The DNA was fragmented using an ultrasonic fragmenter, and then the DNA was fragmented using AMPure XP (AGENCOURT, #A63882) for selection of DNA fragments so that the DNA fragment size was concentrated around 160 bp.

### 3. Library construction:

KAPA HyperPlus Library Preparation Kit (KAPA, #KK8510) was used for library construction, and EZ DNA Metlylation-Gold Kit (Zymo Research, #D5006) was used to perform bisulfite conversion on the adapter ligation product. The specific operation was referred to the kit instructions. The adapters used in the adapter ligation step and the PCR primers used in the PCR step were replaced with adapters and primers suitable for the MGISEQ platform. The adapters needed to be methylated (for example, methylated dCTP was used to synthesize the adapters) to avoid changes in sequence caused by bisulfite treatment.

### 4. High-throughput sequencing:

PE100 sequencing was performed using MGISEQ-2000 (MGI).

### 5. Data analysis

The sequencing data was subjected to quality control, filtration, alignment, and calculation (Non-invasive lung cancer diagnosis and prognosis based on multi-analyte liquid biopsy, Chen Kezhong et al., 2020), and the methylation rate of each CpG site was calculated. By comparing the similarities and differences in the methylation rates of CpG sites in colorectal cancer tissue and para-cancerous tissue, 373 differentially methylated regions (DMRs) between cancer tissue and para-cancerous tissue, i.e., colorectal cancer-specific methylation regions, were identified by the hierarchical Bayes method.

Specific site methylation rate = methylation reads sequencing depth / total sequencing depth.

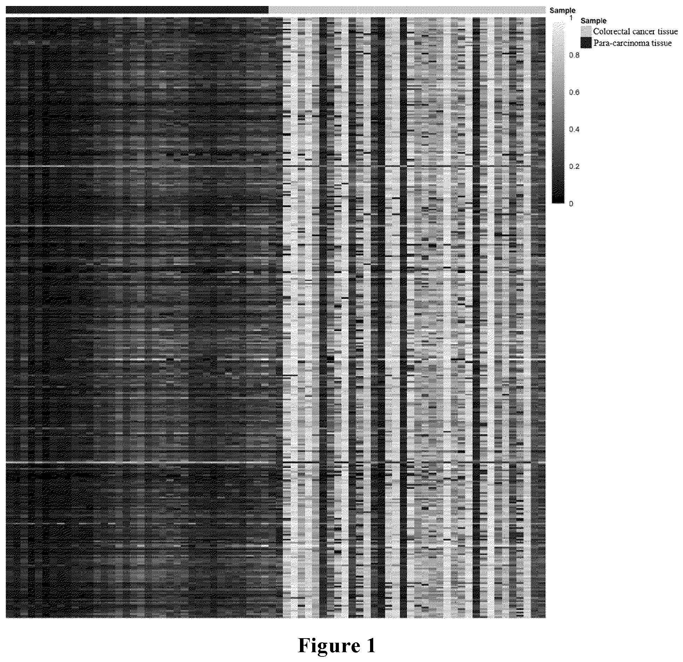

Figure 1 showed a heat map of 373 DMRs found based on 38 pairs of colorectal cancer tissue and para-cancerous tissue, wherein colorectal cancer tissue was on the right and para-cancerous tissue was on the left. The DMRs found included 371 highly methylated DMRs and 2 lowly methylated DMRs. Each grid represented the DMR methylation rate of the corresponding sample at the site, ranging from 0 to 1. The closer the methylation rate was to 0, the darker the color was. The inventors found that the methylation rate difference of the DMR (chr8: 145106171-145107467) of the OPLAH gene was particularly significant.

### Example 2: Screening of colorectal cancer-specific methylated genes based on methylation-specific PCR

In order to further simplify the detection of colorectal cancer-specific methylated genes, methylation-specific primers and probes were designed and screened for the DMR screened in Example 1. It was finally determined that the DMR had high sensitivity and specificity in colorectal cancer detection.

The specific operations were as follows:
1. Candidate genes:
   In the present invention, the differentially methylated regions of the following genes were verified: TFPI2 (chr7: 93519162-93520360), LONRF2 (chr2: 100937780-100939327), ITGA4 (chr2: 182321761-182322766), ZNF132 (chr19: 58951214-58952044), ARHGEF4 (chr2: 131720924-131797968), TLX2 (chr2: 74742305-74743437) and OPLAH (chr8: 145106171-145107467).
2. Design of methylation-specific primers and probes:
   Primers and probes were designed for the above candidate genes. First, the nucleic acid sequence was converted into a sequence after treatment with bisulfite, and then the sites for primer and probe design were determined based on the methylation difference and the detection AUC of the CpG sites, wherein at least one primer in a pair of primers had at least one CpG site at the 3' end.
3. Screening of primers and probes:
   The primers and probes designed in step 2 were used to amplify the methylated standard and non-methylated standard treated with bisulfite, and the primers and probes were screened by evaluating the specificity, accuracy, detection limit and amplification efficiency of the primers and probes. The screened primers and probes for the OPLAH gene DMR were shown in Table 1 above.
4. Performance evaluation of colorectal cancer-specific methylation genes in colorectal cancer detection:
   The primers and probes screened in step 3 were used to detect stool samples from colorectal cancer patients and non-colorectal cancer patients, and the effects of the gene in colorectal cancer detection were compared. First, a nucleic acid extraction kit (BGI) was used to extract fecal DNA, then the DNA was treated with bisulfite, and finally the primers and probes designed above were used to perform fluorescent quantitative PCR detection to obtain Ct value data. The results were treated by SPSS software to draw ROC curves to determine the detection AUC and cutoff value of each gene.

Among them, the internal reference gene primers were set forth in SEQ ID NO: 108 and SEQ ID NO: 109, the OPLAH gene DMR primers were set forth in SEQ ID NO: 29 and SEQ ID NO: 31, the internal reference gene probe was set forth in SEQ ID NO: 110, and the OPLAH gene probe was set forth in SEQ ID NO: 89.

After statistical analysis, the results were shown in Figures 2A to 2G.

The results showed that compared with other candidate genes or regions, the detection of OPLAH gene based on the DMR of the present invention had higher sensitivity and specificity for detecting colorectal cancer, reaching 78.49% and 94.64% respectively, and the AUC was 0.925.

### Example 3: A colorectal cancer detection kit based on the OPLAH gene DMR

This example aimed to develop a colorectal cancer detection kit based on the DMR of OPLAH gene. The performance of OPLAH gene methylation markers for colorectal cancer detection was determined by detecting stool samples from 90 colorectal cancer patients, 98 healthy subjects, and 12 polyp patients.

The specific steps were as follows:
Sample collection: Disposable stool sample samplers (BGI) were used to collect stool samples from the subjects.

Nucleic acid extraction: Stool sample nucleic acid extraction kit (BGI) was used to extract stool sample DNA.

Bisulfite conversion: Methylation detection sample pretreatment kit (BGI) was used to perform C-U conversion on DNA.

qPCR detection: The reaction system comprised 1x PCR Buffer, 2 to 5 mM magnesium ions, 0.2 to 0.8 mM dNTP, 0.2 µM internal reference gene primers (SEQ ID NO: 108, SEQ ID NO: 109), 0.2 µM OPLAH gene primers (SEQ ID NO: 29, SEQ ID NO: 31), 50 nM internal reference gene probe (SEQ ID NO: 110), 50 nM OPLAH gene probe (SEQ ID NO: 89) and 1 unit of Taq polymerase. The reaction comprised pre-denaturation at 95°C for 10min, and then 40 cycles of denaturation at 95°C for 30s, annealing at 55°C for 30s and extension at 72°C for 30s. The detection was performed using Hongshi 96S qPCR instrument with baseline and threshold set as default, and the fluorescence signal was collected at the end of each cycle.

Result analysis: The internal reference gene Ct value was used to evaluate the content of human DNA in the sample. When the Ct value was >37, the human DNA content of the sample was judged to be too low and the detection failed. When the Ct value was ≤37, subsequent analysis could be performed. The positive judgment value of the kit was set to be judged to have high risk of colorectal cancer, when the Ct value of OPLAH gene (represented by DMR) was ≤35 or △Ct (Ct value of OPLAH gene DMR - Ct value of internal reference gene) was ≤5, otherwise it was judged to have low risk. According to the above judgment rules, a total of 66 samples of colorectal cancer patients were detected to have high risk, 89 samples of healthy people were detected to have low risk, and a total of 11 samples of polyps were detected to have low risk. The performance statistics were shown in Table 4.

**Table 4: Detection performance of colorectal cancer detection kit based on OPLAH gene DMR**

| Sample Type | Total number of samples | Prediction results | | Sensitivity for colorectal cancer | Specificity | Specificity of total healthy samples and polyp samples |
|---|---|---|---|---|---|---|
| | | High risk | Low risk | | | |
| Colorectal cancer | 90 | 66 | 24 | 73.33% | / | / |
| Healthy people | 98 | 9 | 89 | / | 90.82% | 90.91% |
| Polyps | 12 | 1 | 11 | / | 91.67% | |

According to the above method, the inventors also used some other primers and probe combinations in Table 1 to test the above samples, and all brought good detection results. The specific results were shown in Table 5 below.

**Table 5**

| Primer sequence No. | Probe sequence No. | Colorectal cancer sensitivity | Specificity |
|---|---|---|---|
| 24, 25 | 83 | 72.22% | 92.73% |
| 25, 26 | 87 | 74.44% | 90.91% |
| 32, 33 | 90 | 78.89% | 84.55% |
| 55, 56 | 103 | 74.44% | 91.82% |

Although the specific embodiments of the present invention have been described in detail, those skilled in the art will understand that various modifications and substitutions can be made to those details based on all the teachings disclosed, and these changes are within the scope of protection of the present invention. The full scope of the present invention is given by the attached claims and any equivalents thereof.

## Claims

1. A differentially methylated region, which is as follows:
chr8: 145106171-145107467.

2. The differentially methylated region according to claim 1, which has a base sequence as set forth in SEQ ID NO: 1 or a complementary sequence thereof.

3. The differentially methylated region according to any one of claims 1 to 2, which is used to detect a colorectal cancer or a colorectal cancer precancerous lesion, or used to perform the prognostic risk assessment of a colorectal cancer patient.

4. The differentially methylated region according to any one of claims 1 to 3, which has undergone the following treatment:
bisulfite treatment, methylation sensitive endonuclease treatment, methylation restriction endonuclease treatment, or methylation modification enzyme treatment.

5. A kit, comprising a reagent for detecting the differentially methylated region according to any one of claims 1 to 4 or a reagent for detecting a methylation site in the differentially methylated region.

6. The kit according to claim 5, wherein the reagent comprises:
(1) a primer and a probe for detecting the differentially methylated region or the methylation site in the differentially methylated region;
preferably, the reagent further comprises:
(2) a primer and a probe for an internal reference gene.

7. The kit according to claim 6, wherein:
in (1), the sequence of the primer is selected from SEQ ID NOs: 2 to 78, and the sequence of the probe is selected from SEQ ID NOs: 79 to 107;
preferably, in (1): the sequence of the primer is selected from SEQ ID NOs: 29 and 31, and the sequence of the probe is selected from SEQ ID NO: 89; the sequence of the primer is selected from SEQ ID NOs: 24 to 25, and the sequence of the probe is selected from SEQ ID NO: 83; the sequence of the primer is selected from SEQ ID NOs: 25 to 26, and the sequence of the probe is selected from SEQ ID NO: 87; the sequence of the primer is selected from SEQ ID NOs: 32 to 33, and the sequence of the probe is selected from SEQ ID NO: 90; or the sequence of the primer is selected from SEQ ID NOs: 55 to 56, and the sequence of the probe is selected from SEQ ID NO: 103;
preferably, in (2), the sequence of the primer of the internal reference gene is set forth in SEQ ID NOs: 108 to 109, and the sequence of the probe of the internal reference gene is set forth in SEQ ID NO: 110.

8. The kit according to any one of claims 5 to 7, further comprising one or more selected from the following:
a stool sample sampler, a nucleic acid extraction reagent, a methylation detection sample pretreatment reagent, and a PCR reagent.

9. The kit according to claim 8, wherein the methylation detection sample pretreatment reagent is a bisulfite, a methylation sensitive endonuclease, a methylation restriction endonuclease, or a methylation modification enzyme.

10. The kit according to any one of claims 5 to 9, further comprising a PCR primer and/or a probe for detecting one or more genes selected from the following:
ZNF132, TLX2, TFPI2, LONRF2, ITGA4, and ARHGEF4.

11. Use of the differentially methylated region according to any one of claims 1 to 4 or a reagent for detecting the differentially methylated region according to any one of claims 1 to 4 or a methylation site in the differentially methylated region in the manufacture of a medicament for detecting a colorectal cancer or a colorectal cancer precancerous lesion, or for assessing the prognostic risk of a colorectal cancer patient.

12. The use according to claim 11, wherein the reagent is a primer and a probe for detecting the differentially methylated region according to any one of claims 1 to 4 or the methylation site in the differentially methylated region.

13. A method for detecting a colorectal cancer or a colorectal cancer precancerous lesion or assessing the prognostic risk of a colorectal cancer patient, comprising the following steps:
detecting the content of the differentially methylated region according to any one of claims 1 to 4 or the content of a fragment thereof; wherein the fragment comprises one or more methylation sites in the differentially methylated region.

14. The method according to claim 13, wherein the content of the differentially methylated region according to any one of claims 1 to 4 or the fragment thereof is detected by a fluorescent quantitative PCR method.

15. The method according to claim 14, wherein,
the content of human DNA in a sample is evaluated by an internal reference gene Ct value, when the internal reference gene Ct value is >37, the sample is judged to have too low human DNA content, and the current detection fails, when the internal reference gene Ct value is ≤37, the sample is evaluated to be qualified and can be analyzed later.

16. The method according to any one of claims 14 to 15, wherein,
if the Ct value of the differentially methylated region is ≤35 or if △Ct ≤ a cutoff value, it is judged to have a high risk of colorectal cancer, otherwise it is judged to have a low risk;
wherein △Ct = (Ct value of the differentially methylated region or Ct value of the amplified fragment) - Ct value of the internal reference gene;
wherein, the amplified fragment comprises one or more methylation sites in the differentially methylated region.

17. The method according to any one of claims 13 to 16, wherein the sample is a human tissue sample, a blood sample, a cell sample, a secretion sample or an excrement sample such as a fecal sample.

18. Use of the differentially methylated region according to any one of claims 1 to 4 in the manufacture of a product for detecting a colorectal cancer or a colorectal precancerous lesion.
